# EUROPEAN PATENT APPLICATION

(11) **EP 4 696 778 A1**
(43) Date of publication of application: **18.02.2026**
(21) Application number: 24836377.2
(22) Date of filing: 05.07.2024
(51) Int. Cl.: C12N 9/24, C12N 1/14, C12R 1/885

(54) **METHOD FOR PRODUCING XYLANASE FROM TRICHODERMA SPP. STRAIN BY USING MN2+ ION**

(30) Priority: 06.07.2023 KR 20230087639
(71) Applicant: CJ Cheiljedang Corporation, Seoul 04560 (KR)
(72) Inventor: KIM, Taekbeom, Seoul 04560 (KR); LEE, Sungkyun, Seoul 04560 (KR); KIM, Young Kyung, Seoul 04560 (KR); LEE, Hyo Hyoung, Seoul 04560 (KR); PARK, Jung Won, Seoul 04560 (KR); KIM, Yu Shin, Seoul 04560 (KR); KIM, Minhoe, Seoul 04560 (KR)
(74) Representative: Meissner Bolte Partnerschaft mbB
(86) International application number: PCT/KR2024/009581
(87) International publication number: WO 2025/009930

(57) **Abstract**

The present invention relates to a method for producing a high concentration of xylanase from a Trichoderma spp. strain, and it was confirmed that, in the fed-batch culture of a *Trichoderma* spp. strain, culturing was performed in a fermentation medium supplemented with Mn²⁺ ions and a feed medium supplemented with Mn²⁺ ions was supplied to the fermentation medium to perform culturing, thereby growing bacterial cells within a short period of time, thus enabling the production of a highly active enzyme. Therefore, the method can be effectively used as a method for mass-producing an enzyme from a *Trichoderma* spp. strain.

## Description

### [TECHNICAL FIELD]

### Cross-Reference to Related Application(s)

This application claims the benefit of priority based on Korean Patent Application No. 10-2023-0087639, filed on July 6, 2023, the entire contents of which are incorporated herein by reference.

The present invention relates to a method for producing high concentrations of xylanase from a *Trichoderma* spp. strain and a medium composition for producing high concentrations of xylanase from a *Trichoderma* spp. strain.

### [BACKGROUND ART]

In the process of manufacturing biofuels and chemical raw materials by saccharifying lignocellulosic raw materials, it is unavoidable that 15 to 30% of xylan contained in the lignocellulosic raw materials is produced as a byproduct. Therefore, the development of a saccharification process for cellulose using cellulase must necessarily be accompanied by xylan utilization technology. Xylan utilization technology involves the production of xylose using xylanase, and what is important in this process is obtaining mass production technology for xylanase having high-titer for efficiently extracting xylan from lignocellulosic raw materials.

As described above, xylanase among various enzymes produced by microorganisms, currently has very high utility, and its various uses are expected to be developed in the future. Currently known uses of xylanase include industrial applications such as the production of high-quality paper and recycling of waste paper, removal of turbid substances from fruit juices and beer, extraction of coffee, vegetable oils and starches, feed additives and the like.

Recently, the utility of xylanase as a feed additive enzyme has increased. Cereal grains for feed, which mainly supply carbohydrates and small amounts of protein required for animal energy metabolism, contain fibrous materials that most animals cannot utilize. Dietary fiber in grains consists of non-starch polysaccharides and lignin, which are components forming the cell walls of plants. Plant cell walls are composed of an outermost layer of cellulose and an inner layer of non-starch polysaccharides such as hemicellulose and beta-glucan, along with lignin, which encapsulate the entire grain. These non-starch polysaccharides are not only indigestible by digestive enzymes of livestock but also encapsulate starch and protein in the grains, thereby reducing the efficiency of nutrient utilization by livestock. Furthermore, in the small intestine, non-starch polysaccharides dissolve into a thick, sticky gel-like substances, which interferes with the access of digestive enzymes to nutrients and inhibits the movement of digesta throughout the digestive tract. Consequently, microorganisms attach to the digesta, thereby causing abnormal fermentation and leading to nutrient loss.

When xylanase is added to grain feed, it can not only increase the availability of nutrients within the grains by hydrolyzing the hemicellulose layer surrounding the grains, but also improve the state of digesta in the animal's intestines. In particular, in case that wheat products with high hemicellulose content are used as animal feed, livestock are prone to develop diarrhea or dysentery, but xylanase can prevent these phenomena, enhance the nutritional value of the feed, and make them a more stable feed ingredient by reducing the variability in the quality of wheat products. Therefore, xylanase is highly valued as a feed additive enzyme. Accordingly, Xylanase is regarded as a highly important feed additive enzyme for animals such as pigs and chickens, and with the recent increase in meat consumption in many countries, diversifying feed resources and improving feed efficiency for livestock production have become especially important issues.

In order to completely degrade and saccharify xylan, which is the main component of hemicellulose, it is generally known that three types of enzymes-endo-xylanase (endo-β-xylanase), exo-xylanase (exo-β-xylanase), and xylosidase (β-xylosidase)-must act together. These enzymes are collectively referred to as xylanase family enzymes.

Microorganisms mainly used for xylanase production include fungi, especially *Trichoderma* spp. strains. These strains have superior enzyme productivity compared to bacteria producing xylanase, and they primarily exhibit maximum activity under acidic conditions, offering the advantage of stability even in the acidic conditions of animal stomachs. Because xylanase for feed additive is an enzyme that is consumed in large quantities at low cost, there is a demand for developing production methods that yield enzymes with high activity and high concentration.

Korean Laid-Open Patent Publication No. 10-2017-0000459 discloses a method for enhancing cellulase activity by co-culturing a *Trichoderma reesei* mutant with *Trichoderma harzianum,* but it does not disclose a method for producing enzyme with high-activity by adding metal ions to the culture medium during the cultivation of *Trichoderma* spp. strain.

### [DISCLOSURE]

### [TECHNICAL PROBLEM]

An object of the present application is to provide a method for producing an enzyme from a *Trichoderma* spp. microorganism, comprising (1) culturing a *Trichoderma* spp. microorganism in a fermentation medium comprising Mn²⁺ ions; and (2) supplying the medium of step (1) with a feed medium comprising Mn²⁺ ions.

Another object of the present application is to provide a composition for xylanase production from a *Trichoderma* spp. strain, comprising a fermentation medium and a feed medium which they comprise Mn²⁺ ions.

### [TECHNICAL SOLUTION]

Specifically, this is explained as follows. Meanwhile, each description and embodiment disclosed in the present application can also be applied to other descriptions and embodiments. That is, all combinations of elements disclosed in the present application fall within the scope of the present application. Furthermore, the scope of the present application should not be considered to be limited by the specific descriptions provided below. In addition, those of ordinary skill in the art will recognize or be able to ascertain many equivalents to the specific aspects of the present application described herein using only routine experimentation. Furthermore, such equivalents are intended to be encompassed by the present application.

An aspect of the present application provides a method for producing an enzyme from a *Trichoderma* spp. microorganism, comprising (1) culturing a *Trichoderma* spp. (genus *Trichoderma*) microorganism in a fermentation medium comprising Mn²⁺ ions; and (2) supplying the medium of step (1) with a feed medium comprising Mn²⁺ ions.

The method for producing an enzyme from a *Trichoderma* spp. microorganism of the present application includes step (1) of culturing a *Trichoderma* spp. microorganism in a fermentation medium comprising Mn²⁺ ions. In the present application, the *Trichoderma* spp. microorganism may be *Trichoderma reesei, Trichoderma viride,* or *Trichoderma harzianum,* and in an embodiment, the *Trichoderma* spp. microorganism may be *Trichoderma reesei.*

In the present application, the enzyme may be xylanase.

In the present application, the culture may be a fed-batch culture.

In the present application, the term "fed-batch culture" refers to a culturing method in which a feed medium is supplied after culturing has begun with a fermentation medium or a basal medium. Fed-batch culture has the advantage that a concentration of substrate in the culture broth can be arbitrarily controlled, and that the substrate is added at an appropriate rate, and does not flow out, allowing for free control over an amount of supplied substrate.

In the present application, the term "medium" refers to a substance mixed with nutrients required for culturing microorganisms as main ingredients, supplying water indispensable for survival and growth, as well as nutrients and growth factors. Specifically, the medium and other culture conditions used for microbial culture in the present application can be used without particular limitation, as long as they are commonly used for microbial culture.

In the present application, the term "fermentation medium" refers to a medium that receives a feed medium (solution of supplying with substrate) in a fed-batch culture method, and may be used interchangeably with the terms "culture medium", "basal medium", "initial medium", or "main medium" herein.

The fermentation medium of step (1) may be a medium comprising Mn²⁺ ions to increase the enzyme production amount from a Trichoderma spp. microorganism or to increase the activity of the enzyme produced from a Trichoderma spp. microorganism.

The concentration of Mn²⁺ ions comprised in the fermentation medium of step (1) may be 1 mM to 12 mM, 2 mM to 12 mM, 3 mM to 12 mM, 4 mM to 12 mM, 1 mM to 11 mM, 2 mM to 11 mM, 3 mM to 11 mM, 4 mM to 11 mM, 1 mM to 10 mM, 2 mM to 10 mM, 3 mM to 10 mM, 4 mM to 10 mM, 1 mM to 9 mM, 2 mM to 9 mM, 3 mM to 9 mM, 4 mM to 9 mM, 1 mM to 8 mM, 2 mM to 8 mM, 3 mM to 8 mM, 4 mM to 8 mM, 1 mM to 7 mM, 2 mM to 7 mM, 3 mM to 7 mM, 4 mM to 7 mM, 1 mM to 6 mM, 2 mM to 6 mM, 3 mM to 6 mM, 4 mM to 6 mM, 4.5 mM to 6 mM, 4.5 mM to 5.5 mM, or 5.0 mM

In the present application, Mn²⁺ ions may be included in the medium in the form of added manganese sulfate (MnSO₄), and in an embodiment, the manganese sulfate may be in the form of manganese sulfate monohydrate (MnSO₄·H₂O), but is not limited thereto.

The fermentation medium of step (1) may comprise manganese sulfate at 0.1 to 1.68 g/L, 0.2 to 1.68 g/L, 0.3 to 1.68 g/L, 0.4 to 1.68 g/L, 0.5 to 1.68 g/L, 0.6 to 1.68 g/L, 0.7 to 1.68 g/L, 0.8 to 1.68 g/L, 0.1 to 1.6 g/L, 0.2 to 1.6 g/L, 0.3 to 1.6 g/L, 0.4 to 1.6 g/L, 0.5 to 1.6 g/L, 0.6 to 1.6 g/L, 0.7 to 1.6 g/L, 0.8 to 1.6 g/L, 0.1 to 1.5 g/L, 0.2 to 1.5 g/L, 0.3 to 1.5 g/L, 0.4 to 1.5 g/L, 0.5 to 1.5 g/L, 0.6 to 1.5 g/L, 0.7 to 1.5 g/L, 0.8 to 1.5 g/L, 0.1 to 1.4 g/L, 0.2 to 1.4 g/L, 0.3 to 1.4 g/L, 0.4 to 1.4 g/L, 0.5 to 1.4 g/L, 0.6 to 1.4 g/L, 0.7 to 1.4 g/L, 0.8 to 1.4 g/L, 0.1 to 1.3 g/L, 0.2 to 1.3 g/L, 0.3 to 1.3 g/L, 0.4 to 1.3 g/L, 0.5 to 1.3 g/L, 0.6 to 1.3 g/L, 0.7 to 1.3 g/L, 0.8 to 1.3 g/L, 0.1 to 1.2 g/L, 0.2 to 1.2 g/L, 0.3 to 1.2 g/L, 0.4 to 1.2 g/L, 0.5 to 1.2 g/L, 0.6 to 1.2 g/L, 0.7 to 1.2 g/L, 0.8 to 1.2 g/L, 0.1 to 1.1 g/L, 0.2 to 1.1 g/L, 0.3 to 1.1 g/L, 0.4 to 1.1 g/L, 0.5 to 1.1 g/L, 0.6 to 1.1 g/L, 0.7 to 1.1 g/L, 0.8 to 1.1 g/L, 0.1 to 1 g/L, 0.2 to 1 g/L, 0.3 to 1 g/L, 0.4 to 1 g/L, 0.5 to 1 g/L, 0.6 to 1 g/L, 0.7 to 1 g/L, 0.8 to 1 g/L, or 0.9 g/L.

The fermentation medium of step (1) may be a medium further comprising Ca²⁺ ions, in addition to Mn²⁺ ions, to increase the amount of enzyme produced from a *Trichoderma* spp. microorganism or to increase the activity of the enzyme produced from *a Trichoderma* spp. microorganism.

The concentration of Ca²⁺ ions comprised in the fermentation medium of step (1) may be 15 to 45 mM, 20 to 45 mM, 22 to 45 mM, 24 to 45 mM, 26 to 45 mM, 28 to 45 mM, 29 to 45 mM, 15 to 40 mM, 20 to 40 mM, 22 to 40 mM, 24 to 40 mM, 26 to 40 mM, 28 to 40 mM, 29 to 40 mM, 15 to 38 mM, 20 to 38 mM, 22 to 38 mM, 24 to 38 mM, 26 to 38 mM, 28 to 38 mM, 29 to 38 mM, 15 to 36 mM, 20 to 36 mM, 22 to 36 mM, 24 to 36 mM, 26 to 36 mM, 28 to 36 mM, 29 to 36 mM, 15 to 34 mM, 20 to 34 mM, 22 to 34 mM, 24 to 34 mM, 26 to 34 mM, 28 to 34 mM, 29 to 34 mM, 15 to 32 mM, 20 to 32 mM, 22 to 32 mM, 24 to 32 mM, 26 to 32 mM, 28 to 32 mM, 29 to 32 mM, 15 to 31 mM, 20 to 31 mM, 22 to 31 mM, 24 to 31 mM, 26 to 31 mM, 28 to 31 mM, 29 to 31 mM, or 30 mM.

In the present application, Ca²⁺ ions may be included in the medium in the form of added calcium chloride (CaCl₂), and in an embodiment, the calcium chloride may be in the form of calcium chloride monohydrate (CaCl₂·H₂O), but is not limited thereto.

The fermentation medium of step (1) may comprise calcium chloride at 0.6 to 5 g/L, 1 to 5 g/L, 1.5 to 5 g/L, 2 to 5 g/L, 2.5 to 5 g/L, 3 to 5 g/L, 3.5 to 5 g/L, 3.6 to 5 g/L, 3.7 to 5 g/L, 0.6 to 4.5 g/L, 1 to 4.5 g/L, 1.5 to 4.5 g/L, 2 to 4.5 g/L, 2.5 to 4.5 g/L, 3 to 4.5 g/L, 3.5 to 4.5 g/L, 3.6 to 4.5 g/L, 3.7 to 4.5 g/L, 0.6 to 4 g/L, 1 to 4 g/L, 1.5 to 4 g/L, 2 to 4 g/L, 2.5 to 4 g/L, 3 to 4 g/L, 3.5 to 4 g/L, 3.6 to 4 g/L, 3.7 to 4 g/L, 0.6 to 3.9 g/L, 1 to 3.9 g/L, 1.5 to 3.9 g/L, 2 to 3.9 g/L, 2.5 to 3.9 g/L, 3 to 3.9 g/L, 3.5 to 3.9 g/L, 3.6 to 3.9 g/L, 3.7 to 3.9 g/L, or 3.8 g/L.

The fermentation medium of step (1) may be a medium including carbohydrates, ammonium sulfate, magnesium sulfate, calcium chloride, manganese sulfate, dipotassium phosphate, yeast extract, ferric sulfate, cobalt chloride, sodium molybdate, and boric acid. Specifically, it may be a medium including 10 to 30 g/L of carbohydrates, 3 to 7 g/L of ammonium sulfate, 1 to 3 g/L of magnesium sulfate, 0.6 to 5 g/L of calcium chloride, 0.1 to 1.68 g/L of manganese sulfate, 3 to 7 g/L of dipotassium phosphate, 5 to 20 g/L of yeast extract, 5 to 15 mg/L of ferric sulfate, 2 to 6 mg/L of cobalt chloride, 0.1 to 4 mg/L of sodium molybdate, and 0.1 to 1 mg/L of boric acid.

The method for producing an enzyme from a *Trichoderma* spp. microorganism of the present application includes step (2) of supplying the medium of step (1) with a feed medium comprising Mn²⁺ ions.

The feed medium of step (2) may be supplied to the fermentation medium when the DO of the fermentation broth increases. The time when DO increases may be when the sugar content is 5 g/L or less.

The feed medium of step (2) may have a supply rate of carbon source comprised in the feed medium of 3 to 7 g/L per hour, 3.5 to 7 g/L, 4 to 7 g/L, 4.5 to 7 g/L, 3 to 6.5 g/L, 3.5 to 6.5 g/L, 4 to 6.5 g/L, 4.5 to 6.5 g/L, 3 to 6 g/L, 3.5 to 6 g/L, 4 to 6 g/L, 4.5 to 6 g/L, 3 to 5.5 g/L, 3.5 to 5.5 g/L, 4 to 5.5 g/L, 4.5 to 5.5 g/L, or 5 g/L.

In the present application, the term "feed medium" refers to a medium that supplies additional carbon sources, nitrogen sources, phosphates, vitamins, and the like to the fermentation medium in a fed-batch culture method, and may be used interchangeably with the term "nutrient medium" herein.

The feed medium of step (2) may be a medium to which Mn²⁺ ions have been added to increase the enzyme production amount from *Trichoderma* spp. microorganism or to increase the activity of the enzyme produced from *Trichoderma* spp. microorganism.

The concentration of Mn²⁺ ions comprised in the feed medium of step (2) may be 6 mM to 14 mM, 7 mM to 14 mM, 8 mM to 14 mM, 9 mM to 14 mM, 6 mM to 13 mM, 7 mM to 13 mM, 8 mM to 13 mM, 9 mM to 13 mM, 6 mM to 12 mM, 7 mM to 12 mM, 8 mM to 12 mM, 9 mM to 12 mM, 6 mM to 11 mM, 7 mM to 11 mM, 8 mM to 11 mM, 9 mM to 11 mM, or 10 mM.

In the present application, Mn²⁺ ions may be included in the feed medium in the form of added manganese sulfate (MnSO₄), but are not limited thereto.

The feed medium of step (2) may comprise manganese sulfate at 1 to 3 g/L, 1.1 to 3 g/L, 1.2 to 3 g/L, 1.3 to 3 g/L, 1.4 to 3 g/L, 1.5 to 3 g/L, 1.6 to 3 g/L, 1.65 to 3 g/L, 1.67 to 3 g/L, 1 to 2.5 g/L, 1.1 to 2.5 g/L, 1.2 to 2.5 g/L, 1.3 to 2.5 g/L, 1.4 to 2.5 g/L, 1.5 to 2.5 g/L, 1.6 to 2.5 g/L, 1.65 to 2.5 g/L, 1.67 to 2.5 g/L, 1 to 2 g/L, 1.1 to 2 g/L, 1.2 to 2 g/L, 1.3 to 2 g/L, 1.4 to 2 g/L, 1.5 to 2 g/L, 1.6 to 2 g/L, 1.65 to 2 g/L, 1.67 to 2 g/L, 1 to 1.8 g/L, 1.1 to 1.8 g/L, 1.2 to 1.8 g/L, 1.3 to 1.8 g/L, 1.4 to 1.8 g/L, 1.5 to 1.8 g/L, 1.6 to 1.8 g/L, 1.65 to 1.8 g/L, 1.67 to 1.8 g/L, 1 to 1.7 g/L, 1.1 to 1.7 g/L, 1.2 to 1.7 g/L, 1.3 to 1.7 g/L, 1.4 to 1.7 g/L, 1.5 to 1.7 g/L, 1.6 to 1.7 g/L, 1.65 to 1.7 g/L, 1.67 to 1.7 g/L, 1.68 to 1.7 g/L, or 1.69 g/L.

The feed medium of step (2) may be a medium further comprising Tween80, in addition to Mn²⁺ ions, to increase the amount of enzyme produced from *Trichoderma* spp. microorganism or to increase the activity of enzyme produced from *Trichoderma* spp. microorganism.

The feed medium of step (2) may comprise Tween 80 at 3 to 10 g/L, 4 to 10 g/L, 5 to 10 g/L, 3 to 9 g/L, 4 to 9 g/L, 5 to 9 g/L, 3 to 8 g/L, 4 to 8 g/L, 5 to 8 g/L, 3 to 7 g/L, 4 to 7 g/L, 5 to 7 g/L, or 6 g/L.

The feed medium of step (2) may be a medium comprising a carbon source and an organic nitrogen source in a weight ratio of 100:0.5 to 5, 100:0.5 to 3, 100:0.5 to 2, or 100:1.

The feed medium of step (2) may be a medium comprising carbohydrates, yeast extract, sodium chloride, manganese sulfate, and Tween 80. Specifically, it may be a medium comprising 500 to 700 g/L of carbohydrates, 2.5 to 25 g/L of yeast extract, 0.1 to 2 g/L of sodium chloride, and 3 to 10 g/L of Tween 80.

The method for producing an enzyme from a *Trichoderma* spp. microorganism of the present application may involve adjusting the concentration of Mn²⁺ ions comprised in the fermentation medium of step (1) and the Mn²⁺ ions comprised in the feed medium of step (2) to increase the amount of enzyme produced from *Trichoderma* spp. microorganism or to increase the activity of the enzyme produced from *Trichoderma* spp. microorganism. Specifically, the concentration ratio of Mn²⁺ ions in the fermentation medium to those in the feed medium may be 1:1 to 1:6, and in an embodiment, it may be 1:2.

The method for producing an enzyme from a *Trichoderma* spp. microorganism, including steps (1) and (2), can increase the activity of the enzyme produced from the *Trichoderma* spp. microorganism, promote the growth of the *Trichoderma* spp. microorganism, and induce the production of the enzyme from the *Trichoderma* spp. microorganism.

The enzyme produced by the method including steps (1) and (2) may have an enzyme activity increased by 15% or more, 20% or more, 25% or more, 30% or more, 35% or more, 40% or more, 45% or more, 47% or more compared to the enzyme produced by culturing in a fermentation medium and feed medium not comprising Mn²⁺ ions, and in an embodiment, it may be increased by 47.8% or more.

The enzyme produced by the method including steps (1) and (2) may have an enzyme activity increased by 15% or more, 20% or more, 25% or more, 26% or more, 27% or more, 28% or more compared to the enzyme produced by culturing in a fermentation medium comprising Mn²⁺ ions and a feed medium not comprising Mn²⁺ ions, and in an embodiment, it may be increased by 28.3% or more.

Additionally, another aspect of the present application provides a composition for xylanase production of a *Trichoderma* spp. strain, comprising a fermentation medium comprising Mn²⁺ ions and a feed medium comprising Mn²⁺ ions.

The *Trichoderma* spp. microorganism, the enzyme, the fermentation medium, the feed medium, Mn²⁺ ions, and the like are as described above

### [ADVANTAGEOUS EFFECTS]

The present invention relates to a method for producing xylanase at a high concentration from a *Trichoderma* spp. strain. It was confirmed that by culturing a *Trichoderma* spp. strain in a fed-batch culture with a fermentation medium comprising Mn²⁺ ions, and supplying a feed medium comprising Mn²⁺ ions to the fermentation medium, enzyme with high-activity could be produced by growing cells in a short period of time. The method can thus be usefully applied as a method for mass producing enzymes from *Trichoderma* spp. strain.

### [BRIEF DESCRIPTION OF THE DRAWING]

FIG. 1 is a drawing representing the cell concentration in the culture solution over time according to the difference in composition of the fermentation medium and the feed medium in fed-batch culture of a *Trichoderma* spp. strain.
FIG. 2 is a drawing representing the activity of produced xylanase over time according to the difference in composition of the fermentation medium and the feed medium in fed-batch culture of a *Trichoderma* spp. strain.
FIG. 3 is a drawing representing the SDS-PAGE results for the protein expression pattern of the culture solution after cultivation, according to the difference in composition of the fermentation medium and the feed medium in fed-batch culture of a *Trichoderma* spp. strain.

### [MODE FOR INVENTION]

Hereinafter, the present invention will be explained in more detail by the following examples. However, these are merely for illustrating the present invention, and the scope of the present invention is not limited by these examples.

### <Preparation of a strain producing xylanase >

For the storage of *Trichoderma reesei* QM6a strain (hereinafter referred to as QM6a strain) deposited under accession number ATCC13631, it was grown on potato dextrose agar plates at 30°C for 5 days. After spore formation, spores were resuspended in 9 g/L of sterile NaCl solution, and 30% sterile glycerol was added. This mixture was stored in 1.8 ml tubes at -80°C.

### <Flask culture method>

QM6a strain was cultured on a potato dextrose agar plate for 7 days, and mycelia were inoculated into 50 ml of potato dextrose broth (PDB) and pre-cultured for 72 hours with shaking at 200 rpm at 30°C. The main culture used a liquid medium composed of 30 g/L lactose, 10 g/L ammonium sulfate, 20 g/L yeast extract, 5 g/L potassium phosphate (KH₂PO₄), and 0.5 g/L magnesium sulfate. 5% (v/v) of the pre-culture solution was inoculated into 200 ml of the liquid medium and cultured for 7 days at 28°C with shaking at 200 rpm, and the culture solution (enzyme solution) was recovered. At this time, the culture temperature was 28°C, and the pH of the liquid medium was 4.0. The recovered culture solution was centrifuged, and the supernatant was used as a crude enzyme solution to measure enzyme activity.

### <Feb-batch culture>

Cultivation of the QM6a strain using fed-batch culture was performed by supplying constantly carbon source in the feed medium at a supplying rate of 5.0 g/L per hour when DO increased, using DO as an indicator. Feed medium was supplied using a peristatic pump. The feed medium was supplied using a peristatic pump. The components of fermentation medium were composed of 20.0 g/L carbohydrate, 5.0 g/L ammonium sulfate, 1.5 g/L magnesium sulfate, 0.5 g/L calcium chloride, 5.0 g/L potassium phosphate, 10.0 g/L yeast extract, 10.0 mg/L ferric sulfate, 4.0 mg/L cobalt chloride, 2.0 mg/L sodium molybdate, 0.4 mg/L boric acid, and 1.0 g/L Tween 80. The feed medium was composed of 600.0 g/L carbohydrate, 6.0 g/L yeast extract, and 1.0 g/L sodium chloride, with a weight ratio of carbon source to yeast extract of 1:0.01. Meanwhile, the fermentation conditions were maintained at a culture temperature of 28°C and ammonia water was used to maintain the pH at 4.0. The stirring speed was adjusted to 600 to avoid DO (dissolved oxygen) limitation. At this time, for the seed culture, 1 ml of frozen spore mixture was inoculated in 150 ml of medium in a 500 ml Erlenmeyer flask, and 10% (v/v) of the culture solution was inoculated by pre-cultivating for 72 hours at 28°C and 200 rpm in a shaker.

### <Method for measuring neutral xylanase activity>

Xylanase activity was measured by quantifying the reducing sugars using the DNS (3,5-dinitrosalicylic acid) assay.

The fermentation broth of *Trichoderma reesei* strain was centrifuged to remove *T. reesei* cells and other solid materials. The culture supernatant was appropriately diluted for enzyme analysis. All enzyme activities were expressed as specific activity in international units (IU) per mL of supernatant. 1(one) IU was defined as an amount of enzyme required to liberate 1 µmol of D-xylose per minute under standard assay conditions (1% xylan, pH 6.5, 50°C).

Under specific temperature and pH conditions, xylanase degrades xylan into oligosaccharides and monosaccharides. Reducing oligosaccharides with terminal reducing ends and monosaccharides possessing reducing groups undergo a colorimetric reaction with the DNS reagent. The color intensity of the reaction solution is proportional to the amount of reducing sugar produced by enzymatic hydrolysis, which in turn is proportional to the activity of xylanase in the reaction solution. Therefore, xylanase activity can be calculated spectrophotometrically.

### EXAMPLE 1. Activity analysis of the enzyme produced from Trichoderma spp. strain upon addition of metal ions to culture medium

To determine whether the addition of various metal ions to the culture medium of *Trichoderma* spp. strains induces production of enzyme with high-activity and to establish optimal conditions of adding metal ion, the following experiments were conducted.

Specifically, various metal ions (Cu²⁺, Mn²⁺, Co²⁺, Zn²⁺, or Fe²⁺) were added to the flask medium used to culture *Trichoderma reesei* QM6a strains at various concentrations (no addition, 1 mM, 10 mM, or 20 mM). Flask cultures were performed using the method described in <Flask Culture Method>. Then, the supernatant from each flask was used as a crude enzyme solution to measure xylanase activity using the method described in <Method for measuring neutral xylanase activity>, and the results are presented in Table 1.

**[Table 1]**

| Metal ions (salts) | Added concentration (mM) | Relative activity of neutral xylanase (%) |
|---|---|---|
| Ca²⁺ (CaCl₂·H₂O) | 0 | 100 |
| | 1 | 104 |
| | 10 | 92 |
| | 20 | 112 |
| Cu²⁺ (CaSO₄) | 0 | 100 |
| | 1 | 168 |
| | 10 | 152 |
| | 20 | 140 |
| Mn²⁺ (MnSO₄·H₂O) | 0 | 100 |
| | 1 | 84 |
| | 10 | 104 |
| | 20 | 172 |
| Co²⁺ (CoCl₂.6H₂O) | 0 | 100 |
| | 1 | 52 |
| | 10 | 104 |
| | 20 | 112 |
| Zn²⁺ (ZnSO₄·7H₂O) | 0 | 100 |
| | 1 | 148 |
| | 10 | 100 |
| | 20 | 96 |
| Fe²⁺ (FeSO₄·7H₂O) | 0 | 100 |
| | 1 | 100 |
| | 10 | 88 |
| | 20 | 116 |

As a result, as shown in Table 1, it was confirmed that the xylanase produced in the medium in which Mn²⁺ ions were added to the *Trichoderma* spp. strain culture medium at a concentration of 20 mM showed a 72% improved enzyme activity compared to the control group (metal ion-free group), demonstrating maximum activity. Furthermore, when Cu²⁺ ions were added at a concentration of 1 mM or 10 mM, and Zn²⁺ ions were added at a concentration of 1 mM, the enzyme activity of xylanase was also improved by 68%, 52%, and 48%, respectively, compared to the control group.

Through the above results, it was confirmed that the addition of Mn²⁺, Cu²⁺ and/or Zn²⁺ to the medium in culturing *Trichoderma* spp. strains could increase the activity of xylanase produced from *Trichoderma* spp. strains.

### Example 2. Confirmation of effect on xylanase production from Trichoderma spp. strain according to addition of metal ions in the fed-batch culture medium

In order to confirm whether the same effect was achieved when the three metal ions Mn²⁺, Zn²⁺, and Cu²⁺ ions, which were confirmed to increase xylanase activity in the flask culture experiment of Example 1, were added to the fed-batch culture medium, the following experiment was performed.

Specifically, as the conditions that enhanced xylanase activity determined in Example 1, Mn²⁺ ions at concentrations of 10 mM and 20 mM, Cu²⁺ ions at concentrations of 1 mM and 10 mM, and Zn²⁺ ions at concentrations of 1 mM, were added to 5 L of fermentation medium of the fed-batch culture, and fed-batch culture was performed according to the method described in the method of <Feb-batch culture>. On the 7th day (168 hours) from the initial culture, the culture solution from each medium was recovered and centrifuged. The supernatant was used as a crude enzyme solution and xylanase activity was measured according to the method described in the <Method for measuring neutral xylanase activity>, and the results are shown in Table 2 below.

**[Table 2]**

| Experimental Condition | Cell Concentration (g/L) | Enzyme Activity (IU/mL) | Comparison of Relative Activity (%) |
|---|---|---|---|
| Control (None) | 240 | 30251 | 0 |
| MnSO₄·H₂O 10 mM | 270 | 37661 | 24.5 |
| MnSO₄·H₂O 20 mM | 260 | 35229 | 16.46 |
| CuSO₄·5H₂O 1 mM | 220 | 29013 | -4.09 |
| CuSO₄·5H₂O 10 mM | 260 | 18626 | -38.43 |
| ZnSO₄·7H₂O 1 mM | 250 | 25163 | -16.82 |

As shown in Table 2, It was confirmed that the xylanase produced from a *Trichoderma* spp. strains showed maximum activity, with enzyme activity improved by 24.5% compared to the control group, when cultured in a fermentation medium supplemented with 10 mM Mn²⁺ ions. When Cu²⁺ ions were added at 1 mM and 10 mM concentrations, xylanase activity decreased by 4% and 38%, respectively, and when Zn²⁺ ions were added at 1 mM concentration, enzyme activity also decreased by 16.8%.

Through the above results, it was confirmed that addition of Mn²⁺ ions to the fermentation medium in the fed-batch culture of *Trichoderma* spp. strains was the optimal condition for increasing the activity of the produced xylanase.

### Example 3. Confirmation of the optimal concentration of Mn²⁺ concentration for the high-concentration production of xylanase from Trichoderma spp. strain

Example 2 confirmed that adding Mn²⁺ ions to the fermentation medium in fed-batch culture of *Trichoderma* spp. strains was the optimal condition for increasing the activity of the produced xylanase. The following experiment was performed to determine the optimal concentration of Mn²⁺ ion to be added to the fermentation medium of fed-batch culture for high-concentration production of xylanase from *Trichoderma* spp. strains.

Specifically, except that Mn²⁺ ions were added to the fermentation medium at concentrations of 5, 10, 15, 20, and 30 mM, respectively, the cultivation was performed for 7 days from the initial cultivation date using the same medium composition and fermentation method described in the method of <Feb-batch culture>. The supernatant obtained by centrifuging the culture solution, was used as a crude enzyme solution. Xylanase activity was measured using the method described in the <Method for measuring neutral xylanase activity>, and is presented in Table 3 below.

**[Table 3]**

| Experimental Condition | Cell Concentration (g/L) | Enzyme Activity (IU/mL) | Comparison of Relative Activity (%) |
|---|---|---|---|
| Control (None) | 245 | 33552 | 0 |
| MnSO₄·H₂O (Main 5 mM) | 291 | 34488 | 2.79 |
| MnSO₄·H₂O (Main 10 mM) | 250 | 39992 | 19.19 |
| MnSO₄·H₂O (Main 15 mM) | 260 | 38604 | 15.06 |
| MnSO₄·H₂O (Main 20 mM) | 235 | 36924 | 10.05 |
| MnSO₄·H₂O (Main 30 mM) | 205 | 30336 | -9.59 |

As a result, as shown in Table 3, the xylanase produced from the *Trichoderma* spp. strain showed maximum activity when Mn²⁺ ions were added to the fermentation medium (main medium) at a concentration of 10 mM. Specifically, when Mn²⁺ ions were added at a concentration of 10 mM, the enzyme activity was enhanced by 19.6% compared to the control group, and when Mn²⁺ ions were added at a concentration of 20 mM or higher, the cell concentration decreased and the enzyme activity also showed a decreasing pattern.

By reflecting the above results, the components of the fermentation medium for the *Trichoderma* spp. strain were adjusted to 20.0 g/L of carbohydrate, 5 g/L of ammonium sulfate, 1.5 g/L of magnesium sulfate, 0.5 g/L of calcium chloride, 1.51 g/L of manganese sulfate (anhydrate basis), 5.0 g/L of potassium phosphate dibasic, 10.0 g/L of yeast extract, 10 mg/L of ferric sulfate, 4.0 mg/L of cobalt chloride, 2.0 mg/L of sodium molybdate, and 0.4 mg/L of boric acid, and the feed medium was adjusted to 600 g/L of carbohydrate, 6.0 g/L of yeast extract, and 1.0 g/L of sodium chloride, with a weight ratio of the carbon source and yeast extract of 1:0.01. This was referred to as the adjusted medium of Example 3 (hereinafter, the adjusted medium of Example 3).

### Example 4. Confirmation of supplying method of metal ions for fed-batch culture for the high-concentration production of xylanase from Trichoderma spp. strain

Fed-batch culture is a microbial cultivation method that regulates the supply of fresh culture medium, growth-limiting substrate solutions, or growth-limiting nutrients such as amino acids, vitamins, phosphate, nitrogen sources, trace metal ions, and carbon sources during batch culture. This method maintains nutrient concentrations at appropriate levels throughout the culture period. In this fed-batch culture mode, the following experiments were conducted to determine the optimal supply method of metal ion by varying the supply method of Mn²⁺ ions, the metal ions determined in Examples 2 and 3, and analyzing each concentrations of metal ions, which inhibited cell mass and enzyme activity.

Specifically, except that the Mn²⁺ ion concentration in the fermentation medium (culture medium or main medium) in the fed-batch mode was set to 5 mM or 10 mM, and the Mn²⁺ ion concentration in the feed medium was added to 5 mM or 10 mM, respectively, the cultivation was performed for 7 days from the initial cultivation date using the same medium composition and fermentation method described in the method of <Feb-batch culture>. The supernatant obtained by centrifuging the culture solution, was used as a crude enzyme solution. Xylanase activity was measured using the method described in the <Method for measuring neutral xylanase activity>, and is presented in Table 4 below.

**[Table 4]**

| Experimental Condition | Cell Concentration (g/L) | Enzyme Activity (IU/mL) | Comparison of Relative Activity (%) |
|---|---|---|---|
| Adjusted medium of Example 3 | 235 | 38652 | 0 |
| MnSO₄·H₂O (Feed 10 mM) | 210 | 39787 | 1.03 |
| MnSO₄·H₂O (Main 5 mM, Feed 5 mM) | 235 | 47027 | 21.67 |
| MnSO₄·H₂O (Main 5 mM, Feed 10 mM) | 232 | 49601 | 28.33 |
| MnSO₄·H₂O (Main 10 mM, Feed 5 mM) | 281 | 47991 | 24.16 |
| MnSO₄·H₂O (Main 10 mM, Feed 10 mM) | 272 | 45075 | 16.62 |

As a result, as shown in Table 4, it was confirmed that the method of supplying Mn²⁺ ions in a constant ratio to the feed medium increased the activity of xylanase produced from *Trichoderma* spp. strains in the fed-batch mode, rather than the method of supplying all Mn²⁺ ions at once to the fermentation medium. The maximum activity was achieved in the fermentation medium and feed medium at a ratio of 1:2, in which the method of supplying Mn²⁺ ions at a concentration of 5 mM to the culture medium and 10 mM to the feed medium, showed the best improvement in enzyme activity by 28.33%.

By reflecting the above results, the components of the fermentation medium for the *Trichoderma* spp. strain were adjusted to carbohydrate 20.0 g/L, ammonium sulfate 5 g/L, magnesium sulfate 1.5 g/L, calcium chloride 0.5 g/L, manganese sulfate 0.76 g/L (anhydrous basis), potassium phosphate 5.0 g/L, yeast extract 10.0 g/L, ferric sulfate 10 mg/L, cobalt chloride 4.0 mg/L, sodium molybdate 2.0 mg/L, and boric acid 0.4 mg/L, and the feed medium was adjusted to carbohydrate 600 g/L, yeast extract 6.0 g/L, sodium chloride 1.0 g/L, manganese sulfate 1.51 g/L (anhydrous basis), and Tween 80 1.0 with a weight ratio of carbon source and yeast extract of 1:0.01. g/L. This was referred to as the adjusted medium of Example 4 (hereinafter, adjusted medium of Example 4).

### Example 5. Confirmation of effect of increasing xylanase activity from Trichoderma spp. strain according to increased amount of Ca²⁺ ions in medium

In consideration that calcium channels are involved in the xylanase production (secretion) process in *Trichoderma reesei* strains, the following experiments were performed to determine the effect of increasing xylanase production according to an additional increase in concentration of Ca²⁺ ion.

Specifically, except that 10 mM, 30 mM, or 50 mM of Ca²⁺ ions were added to the fermentation medium of the adjusted medium described in Example 4, or 10 mM of Ca²⁺ ions was added to the fermentation medium and 10 mM of Ca²⁺ ions was added to the feed medium, the cultivation was performed for 7 days from the initial cultivation date using the same medium composition and fermentation method described in the method of <Feb-batch culture>. The supernatant obtained by centrifuging the culture solution, was used as a crude enzyme solution. Xylanase activity was measured using the method described in the <Method for measuring neutral xylanase activity>, and is presented in Table 5 below.

**[Table 5]**

| Experimental Condition | Cell Concentration (g/L) | Enzyme Activity (IU/mL) | Comparison of Relative Activity (%) |
|---|---|---|---|
| Adjusted medium of Example 4 | 252 | 45720 | 0 |
| CaCl₂ (Main 10 mM) | 254 | 49975 | 9.3 |
| CaCl₂ (Main 30 mM) | 248 | 51720 | 13.1 |
| CaCl₂ (Main 10 mM, Feed 10 mM) | 266 | 48665 | 6.4 |
| CaCl₂ (Main 15 mM, Feed 15 mM) | 242 | 48393 | 5.8 |

As a result, as shown in Table 5, as the concentration of Ca²⁺ ions added to the fermentation medium of fed-batch increased, the activity of xylanase produced from the *Trichoderma* spp. strain increased proportionally, and when Ca²⁺ ions were added to the fermentation medium at a concentration of 30 mM, the maximum xylanase activity was achieved. On the other hand, unlike the case of adding Mn²⁺ ions, it was confirmed that the method of supplying Ca²⁺ ions with splitting them in a certain ratio to the fermentation medium and the feed medium actually decreased the effect of improving enzyme activity.

By reflecting the above results, the components of the fermentation medium for *Trichoderma* spp. strains were adjusted to 20.0 g/L carbohydrate, 5 g/L ammonium sulfate, 1.5 g/L magnesium sulfate, 3.8 g/L calcium chloride, 0.9 g/L manganese sulfate, 5.0 g/L potassium phosphate, 10.0 g/L yeast extract, 10 mg/L ferric sulfate, 4.0 mg/L cobalt chloride, 2.0 mg/L sodium molybdate, and 0.4 mg/L boric acid, and the feed medium was adjusted to 600 g/L carbohydrate, 6.0 g/L yeast extract, 1.0 g/L sodium chloride, 1.51 g/L manganese sulfate (anhydrate basis), and 1.0 g/L Tween 80, with a weight ratio of carbon source and yeast extract of 1:0.01. This was referred to as the adjustment medium of Example 5 (hereinafter, adjustment medium of Example 5).

### Example 6. Confirmation of effect of increasing xylanase activity from Trichoderma spp. strain according to increased amount of surfactant in medium

Surfactants contained in the medium enhance intracellular calcium signaling and enzyme release by loosening cell walls or inducing mycelial dispersion. To determine whether increasing the amount of surfactant (Tween 80) in the medium in a fed-batch culture mode of *Trichoderma* spp. strains improves xylanase activity, the following experiment was conducted.

Specifically, except that Tween 80 was added to the feed medium in the conditioned medium of Example 5 at concentrations of 1.0 g/L, 5.0 g/L, and 10.0 g/L, respectively, the cultivation was performed for 7 days from the initial cultivation date using the same medium composition and fermentation method described in the method of <Feb-batch culture>. The supernatant obtained by centrifuging the culture solution, was used as a crude enzyme solution. Xylanase activity was measured using the method described in the <Method for measuring neutral xylanase activity>, and is presented in Table 6 below.

**[Table 6]**

| Experimental Condition | Cell Concentration (g/L) | Enzyme Activity (IU/mL) | Comparison of Relative Activity (%) |
|---|---|---|---|
| Adjusted medium of Example 5 | 280 | 51730 | 0 |
| Tween80 1.0 g/L | 278 | 52790 | 2 |
| Tween80 5.0 g/L | 325 | 112160 | 116.8 |
| Tween80 10.0 g/L | 290 | 77360 | 49.5 |

As a result, as shown in Table 6, when Tween80 was additionally added at a concentration of 5.0 g/L to the feed medium of fed-batch culture, the activity of xylanase produced from *Trichoderma* spp. strains increased to the maximum. Compared to when the amount of Tween80 in the feed medium was included at a concentration of 1.0 g/L without increase in the amount of Tween 80, the activity of xylanase produced was significantly improved by 116.8%.

By reflecting the above results, the components of the fermentation medium for *Trichoderma* spp. strains were adjusted to 20.0 g/L carbohydrate, 5 g/L ammonium sulfate, 1.5 g/L magnesium sulfate, 3.8 g/L calcium chloride, 0.9 g/L manganese sulfate, 5.0 g/L potassium phosphate, 10.0 g/L yeast extract, 10 mg/L ferric sulfate, 4.0 mg/L cobalt chloride, 2.0 mg/L sodium molybdate, and 0.4 mg/L boric acid, and the feed medium was adjusted to 600 g/L carbohydrate, 6.0 g/L yeast extract, 1.0 g/L sodium chloride, 1.51 g/L manganese sulfate, and 6.0 g/L Tween80, with a weight ratio of carbon source and yeast extract of 1:0.01. This was referred to as the adjusted medium of Example 6 (hereinafter, adjusted medium of Example 6).

### Experimental Example 1. Comparison of xylanase activities and protein expression levels according to various medium compositions

### Experimental Example 1-1. Confirmation of the change in microbial cell concentrations according to various medium compositions

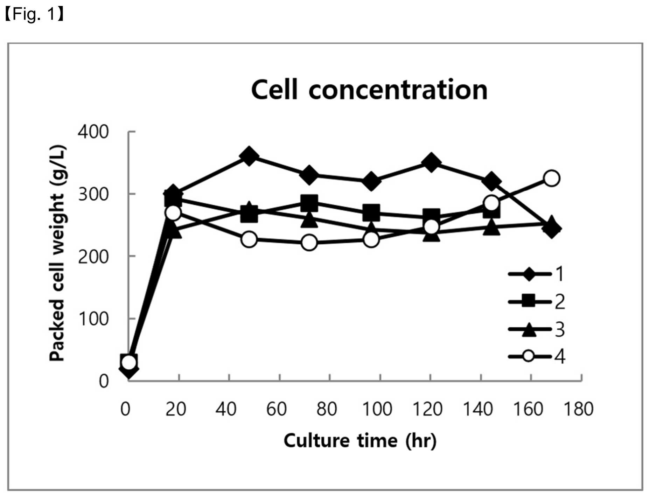

In order to determine the growth pattern of *Trichoderma* spp. strains according to the various medium compositions obtained from the Examples, *Trichoderma reesei* QM6a strain was cultured in the following medium compositions: a control group with no addition of metal ions (indicated as 1 in the figure), experimental group 1 (indicated as 2 in the figure) with addition of 5 mM of MnSO₄·H₂O to the fermentation medium and 10 mM of MnSO₄·H₂O to the feed medium; experimental group 2 (indicated as 3 in the figure) with addition of 5 mM of MnSO₄·H₂O to the fermentation medium,10 mM of MnSO₄·H₂O to the feed medium and additional addition of 30 mM of CaCl₂ to the fermentation medium; and experimental group 3 (indicated as 4 in the figure) with addition of 5 mM of MnSO₄·H₂O to the fermentation medium and 10 mM of MnSO₄·H₂O to the feed medium, and additional addition of 30 mM of CaCl₂ to the fermentation medium, and additional addition of 5 g/L of Tween80 to the feed medium. Then, the changes in cell concentration in each culture solution over time were observed and shown in Fig. 1.

As a result, as shown in Fig. 1, it was confirmed that the cell mass was maintained somewhat low in the early stage of cultivation, but the cell mass increased again in the latter stage of fermentation.

### Experimental Example 1-2. Comparison of relative activities of xylanase according to various medium compositions

To compare the relative activities of xylanase produced from *Trichoderma* spp. strains according to each medium composition determined in Examples 3 to 6, the enzyme activities measured in each Example were compared and shown in Table 7 below.

**[Table 7]**

| Experimental Condition | Cell Concentration (g/L) | Enzyme Activity (IU/mL) | Comparison of Relative Activity(%) |
|---|---|---|---|
| Control(None) | 245 | 33552 | 0 |
| Medium composition of Example 3 | 250 | 39992 | 19.2 |
| Medium composition of Example 4 | 232 | 49601 | 47.8 |
| Medium composition of Example 5 | 270 | 54120 | 61.3 |
| Medium composition of Example 6 | 325 | 112160 | 234.3 |

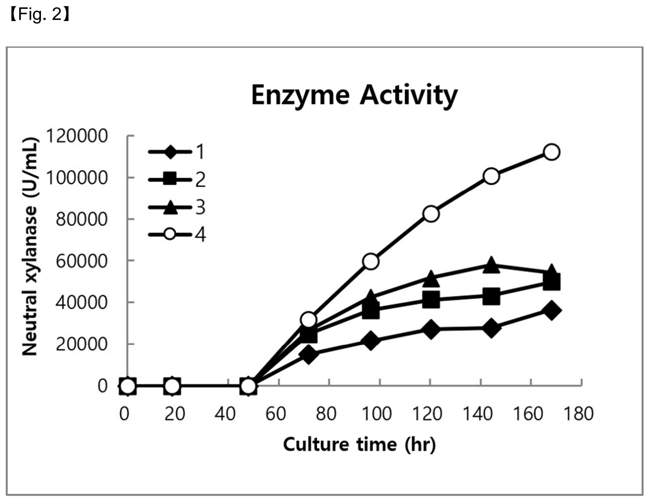

In addition, *Trichoderma reesei* QM6a strain was cultured in the following medium compositions: a control group without metal ions (indicated as 1 in the figure), experimental group 1 (indicated as 2 in the figure) with addition of 5 mM of MnSO₄·H₂O to the fermentation medium and addition of 10 mM MnSO₄ to the feed medium, experimental group 2 (indicated as 3 in the figure) with addition of 5 mM MnSO₄·H₂O to the fermentation medium and addition of 10 mM MnSO₄·H₂O to the feed medium, and additional addition of 30 mM CaCl₂ to the fermentation medium, and experimental group 3 (indicated as 4 in the figure) with addition of 5 mM MnSO₄·H₂O to the fermentation medium and addition of 10 mM to the feed medium, additional addition of 30 mM CaCl₂ to the fermentation medium, and additional addition of 5 g/L Tween80 to the feed medium. Then, the xylanase activity in each culture solution over time were observed and shown in Fig. 2.

As a result, as shown in Fig. 2 and Table 7, when (1) 5 mM of MnSO₄·H₂O was added to the fermentation medium and 10 mM of MnSO₄·H₂O was added to the feed medium, (2) 30 mM of CaCl₂ was additionally added to the fermentation medium, and (3) 5 g/L of Tween80 was additionally added to the feed medium, during fed-batch cultivation of *Trichoderma* spp. Strain, it was confirmed that the activity of xylanase produced from the strain was significantly improved by 234.3% compared to the control group.

### Experimental Example 1-3. Comparison of protein expression level using optimal medium compositions

To determine changes in target protein expression levels from *Trichoderma* spp. strains according to medium composition, the following SDS-PAGE analysis experiment was performed.

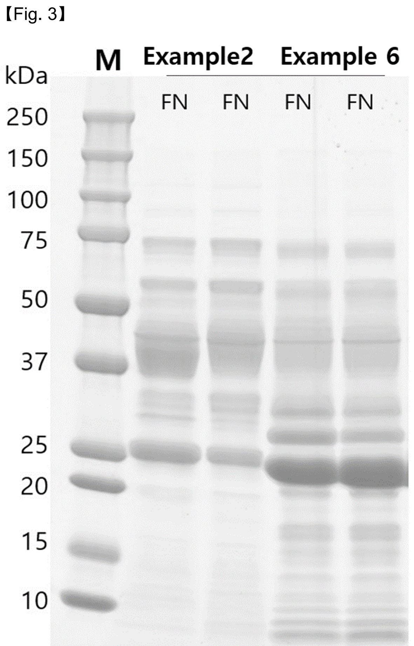

Specifically, *Trichoderma reesei* QM6a strain was cultured in a control medium with no addition of metal ions and in a medium composition of Example 6 that aMn²⁺ ions, Ca²⁺ ions, and Tween 80 were added. Then, the culture solutions were centrifuged at 25°C and 10,000 rpm for 20 minutes to separate the cells and supernatant. 40 µl of the separated supernatant was mixed with 10 µl of 5X staining buffer and subjected to SDS-PAGE (10%) to determine the protein mobility according to molecular weight. After electrophoresis, the polyamide gel was stained with Coomassie Brilliant R250 to determine the composition and molecular weight of the proteins. The results are shown in Fig. 3.

As a result, as shown in Fig. 2, it was confirmed that the expression level of the 25 kDa protein corresponding to the target protein of neutral xylanase, increased in the culture solution cultured in the medium composition of Example 6.

From the above description, those skilled in the art will understand that the present application can be implemented in other specific forms without altering the technical concept or essential features thereof. In this regard, it should be understood that the embodiments described above are illustrative in all respects and not restrictive. The scope of this application should be interpreted to include all changes or modifications derived from the meaning and scope of the following claims and their equivalents, rather than the detailed description above.

## Claims

1. A method for producing an enzyme from *Trichoderma* spp. microorganism, comprising
(1) culturing *Trichoderma* spp. microorganisms in a fermentation medium comprising Mn²⁺ ions; and
(2) supplying the medium of step (1) with a feed medium comprising Mn²⁺ ions.

2. The method for producing an enzyme according to claim 1, wherein the *Trichoderma* spp. microorganism is *Trichoderma reesei.*

3. The method for producing an enzyme according to claim 1, wherein the enzyme is xylanase.

4. The method for producing an enzyme according to claim 1, wherein a concentration of Mn²⁺ ions comprised in the fermentation medium of step (1) is 1 mM to 12 mM.

5. The method for producing an enzyme according to claim 1, wherein a concentration of Mn²⁺ ions comprised in the feed medium of step (2) is 6 mM to 14 mM.

6. The method for producing an enzyme according to claim 1, wherein the feed medium of step (2) is supplied to the fermentation medium when the dissolved oxygen (DO) level in the fermentation broth increases.

7. The method for producing an enzyme according to claim 1, wherein a supplying rate of a carbon source in the feed medium of step (2) is 3.0 to 7.0 g/L per hour.

8. The method for producing an enzyme according to claim 1, wherein the fermentation medium of step (1) further comprises Ca²⁺ ions.

9. The method for producing an enzyme according to claim 8, wherein a concentration of the Ca²⁺ ions is 15 mM to 45 mM.

10. The method for producing an enzyme according to claim 1, wherein the feed medium of step (2) comprises Tween80.

11. The method for producing an enzyme according to claim 10, wherein a concentration of Tween80 is 3 to 10 g/L.

12. A composition for producing xylanase from *Trichoderma* spp. strain, comprising a fermentation medium and a feed medium which comprise Mn²⁺ ions.

13. The composition for producing xylanase according to claim 12, wherein a concentration of Mn²⁺ ions comprised in the fermentation medium of the composition is 1 mM to 12 mM.

14. The composition for producing xylanase according to claim 12, wherein a concentration of Mn²⁺ ions comprised in the feed medium of the composition is 6 mM to 14 mM.

15. The composition for producing xylanase according to claim 12, wherein the fermentation medium of the composition further comprises Ca²⁺ ions.

16. The composition for producing xylanase according to claim 15, wherein a concentration of Ca²⁺ ions comprised in the fermentation medium is 15 mM to 45 mM.

17. The composition for producing xylanase according to claim 12, wherein the feed medium of the composition comprises Tween80.

18. The composition for producing xylanase according to claim 17, wherein a concentration of Tween80 comprised in the feed medium is 3 to 10 g/L.
